(19) Europäisches Patentamt
European Patent Office
Office européen des brevets

(11) Numéro de publication: **0 012 079**
**B1**

# (12) FASCICULE DE BREVET EUROPEEN

(45) Date de publication du fascicule du brevet: **29.09.82**

(21) Numéro de dépôt: **79400926.6**

(22) Date de dépôt: **28.11.79**

(51) Int. Cl.[3]: **C 07 D 491/107,**
C 07 D 513/10,
G 03 C 1/733 //C07D211/70,
C07D279/12

## (54) Composés spiropyranniques des cycles pipéridine ou thiazine, procédé de préparation et application dans les compositions photochromes.

(30) Priorité: **28.11.78 FR 7833512**

(43) Date de publication de la demande:
**11.06.80 Bulletin 80/12**

(45) Mention de la délivrance du brevet:
**29.09.82 Bulletin 82/39**

(84) Etats contractants désignés:
**CH DE GB IT**

(56) Documents cités:
**FR - A - 2 319 643**
**COMPTES RENDUS DES SEANCES HEBDOMADAIRES DE L'ACADEMIE DES SCIENCES, serie C, Sciences chimiques, vol 282, 16 mai 1976, Paris, FR, M. MAGUET et al.: "Synthèse de composés photochromes en série azahétérocyclique non condensée. Spiropyrannes tétrahydro 2.4.5.6 oxaziniques 1,3" pages 943—945**

(73) Titulaire: **ETAT-FRANCAIS représenté par le DELEGUE GENERAL POUR L'ARMEMENT**
**Bureau des Brevets et Inventions de la Délégation Générale pour l'Armement 14, rue Saint-Dominique**
**F-75997 Paris Armées (FR)**

(72) Inventeur: **Guglielmetti, Robert J.**
**1 rue Maurice Donnay**
**F-29200 Brest (FR)**
Inventeur: **Garnier, Francis**
**17, Villa Rémy**
**F-94000 Champigny (FR)**
Inventeur: **Poirier, Yves M.**
**75, rue Commandant Groix**
**F-29200 Brest (FR)**
Inventeur: **Petillon, Gisèle M.C.**
**Raudaufloc'h Edem**
**F-29112 Briec (FR)**

(56) Documents cités
**JOURNAL OF HETEROCYCLIC CHEMISTRY, vol. 15, no. 8, decembre 1978, Provo, Utah, US, M. MAGUET et al.: "Réactivité de spirochromènes et de mérocyanines azahéterocycliques vis-à-vis de quelques agents nucleophiles", pages 1439—1446**

**EP 0 012 079 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

Courier Press, Leamington Spa, England.

Composés spiropyranniques des cycles pipéridine ou thiazine, procédé de préparation et application dans les compositions photochromes

Le secteur technique de la présente invention est celui des composés spiropyranniques utilisables dans l'enregistrement d'informations notamment par voie optique ou thermique.

Un certain nombre de composés spiropyranniques doués de propriétés photochromes ont été proposés dans la littérature et il est connu que ces composés se transforment sous l'effet des photons en des mérocyanines colorées.

Dans le brevet français 2 008 056 par exemple, on a proposé des spiropyrannes indoliniques utilisables pour la photographie en couleur avec un support constitué par l'acétate de polyvinyle et dont le maximum d'absorption se situe autour de 570 nm.

Dans le brevet français 2 105 021, on décrit des dérivés spiropyranniques benzothiazoliniques doués de propriétés photochromes dans une gamme de longueurs d'onde allant de 215 à 350 nm.

Le compte rendu de l'Académie des Sciences de Paris 1.282 (17 mai 1976) divulgue des spiropyrannes tétrahydro-2.4.5.6. Oxaziniques-1.3 doués de propriétés photochromes. Cependant, aucune longueur d'onde d'absorption n'est indiquée.

Le brevet français 2 319 643 décrit des composés spiropyranniques doués de propriétés photochromes dans lesquels l'hétérocycle contenant l'azote et le soufre ou l'oxygène comporte cinq chaînons. Le domaine des longueurs d'onde d'absorption n'est pas précisé.

L'objectif de la présente invention est de mettre à la disposition de l'homme de l'art de nouveaux composés doués de remarquables propriétés photochromes et dont les propriétés spectrocinétiques sont plus avantageuses que celles des composés de l'art antérieur.

L'invention a pour objet de nouveaux composés spiropyranniques, caractérisés en ce qu'ils répondent à la formule générale (I)

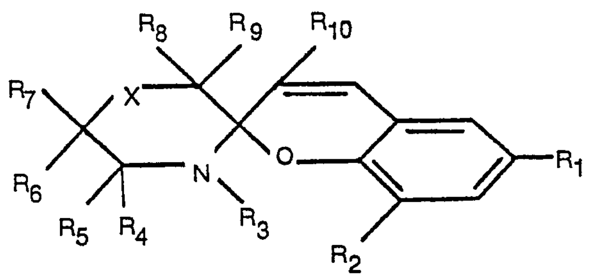

(I)

dans laquelle:

X représente un groupement $CH_2$ ou un atome de soufre:

$R_1$ et $R_2$ représentent des groupements accepteurs et/ou donneurs d'électrons, $R_3$ est un groupement alkyle, linéaire ou ramifié, comportant de 1 à 18 atomes de carbone, un groupement phénylalkyle dans lequel le groupe alkyle linéaire ou ramifié comporte de 1 à 5 atomes de carbone, un groupement alcényle comportant 2 à 10 atomes de carbone;

$R_4$, $R_5$, $R_6$, $R_7$, $R_8$ et $R_9$ présentent l'une quelconque des significations indiquées pour $R_3$, représentent un atome d'hydrogène, ou participent deux à deux à la formation d'un homocycle saturé de 5 à 10 atomes de carbone, soit avec le groupement se trouvant sur le même atome de carbone, soit avec le groupement en ortho;

$R_{10}$ représente si $X = S$ un atome d'hydrogène et si $X = CH_2$ un atome d'hydrogène un groupement alkyle comportant 1 à 18 atomes de carbone, un groupement alcényle comportant 2 à 10 atomes de carbone.

Les caractéristiques suivantes peuvent être utilisées:

— $R_1$ et $R_2$ sont choisis dans le groupe constitué par: l'hydrogène $NO_2$, —CN, les halogènes, les substituants —$COC_6H_5$, méthoxy, les groupements alkyle, mercaptoalkyle, les éthers de formule —$CH_2$ OR, des thioéthers de formule $CH_2$ SR et des amines de formule —$CH_2NR_2$ dans lesquelles R représente un groupe alkyle; $R_3$ est choisi parmi les substituants méthyle, éthyle, isopropyle, n-butyle, benzyle ou phénéthyle; $R_4$, $R_5$, $R_6$, $R_7$, $R_8$ et $R_9$ présentent l'une des significations indiquées pour $R_3$, ou représentent deux à deux avec le groupement en ortho un groupe —$(CH_2)$ n- dans lequel n est un nombre compris entre 5 et 10, ou un atome hydrogène; et $R_{10}$ représente si $X = S$ un atome hydrogène et si $X = CH_2$ un atome d'hydrogène, un substituant méthyle ou éthyle.

— $R_1$ est représenté par $NO_2$, $OCH_3$, $COC_6H_5$, I ou Br; $R_2$ est représenté par $OCH_3$, $NO_2$, H, I ou Br; $R_4$ à $R_9$ sont représentés par un atome d'hydrogène et un groupement méthyle, éthyle, isopropyle, n-butyle, benzyle ou phénétyle et $R_{10}$ représente si $X = S$ un atome d'hydrogène et si $X = CH_2$ un atome d'hydrogène, un substituant méthyle ou éthyle.

— $R_3$ est représenté par un groupement méthyle, éthyle ou isopropyle; $R_4$ à $R_9$ son choisis dans le groupe constitué par l'hydrogène et les groupements méthyle, éthyle ou isopropyle.

— X représente un groupement $CH_2$, $R_4$ et $R_5$ des atomes d'hydrogène, et $R_8$ et $R_9$ des substituants méthyle et $R_{10}$ un substituant méthyle ou éthyle.

— X représente un atome de soufre, $R_6$ et $R_7$ des atomes d'hydrogène et $R_8$ et $R_9$ des substituants méthyle et $R_{10}$ l'hydrogène.

L'invention concerne également un procédé de préparation de dérivés spiropyranniques, caractérisé par le fait qu'il comprend la condensation d'un aldéhyde aromatique orthohydroxylé de formule (III) avec un sel quaternaire de formule (IV):

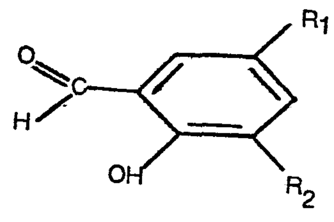

(III)

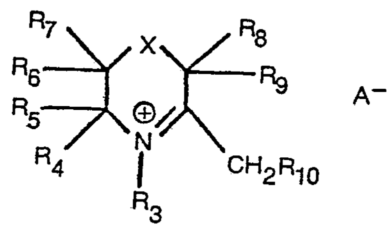

(IV)

dans lesquelles X et $R_1$ à $R_{10}$ ont les significations précédemment indiquées et A représente un anion choisi dans le groupe des anions iodure, tosylate, méthylsulfate.

La condensation est préférentiellement réalisée en milieu basique.

Le sel quaternaire de formule (IV), est obtenu en faisant réagir un agent d'alkylation de formule (V) sur une base de formule (VI) selon la réaction:

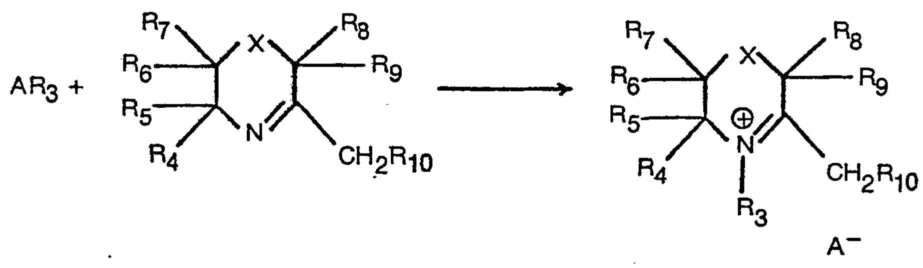

(V) (VI) (IV)

où $R_3$ à $R_{10}$, X et A présentent les significations sus-indiquées.

Pour obtenir les produits dans lesquels X représente un groupement $CH_2$, on prépare la $\Delta$ 1-pipéridéine de formule (VI) par condensation de $LiCH_2$ $R_{10}$ sur un nitrile $\omega$-halogéné de formule VIII selon le schéma:

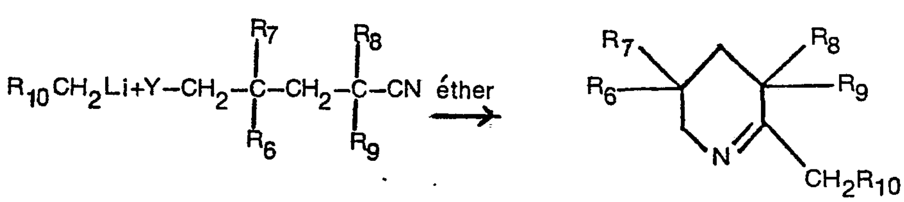


(VII) (VIII) (VI)

les groupements $R_6$ à $R_{10}$ présentant les significations indiquées et y le chlore ou le brome.

Pour obtenir les produits dans lesquels X représente un atome de soufre, on condense une mercaptocétone de formule (IX) sur une aziridine de formule (X), selon le schéma:

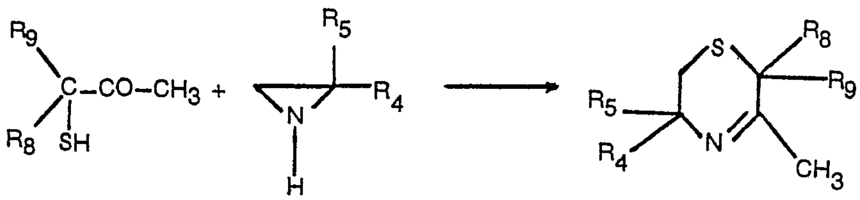

(IX) (X) (VI)

$R_4$, $R_5$, $R_8$ et $R_9$ ayant les significations sus-indiquées.

L'invention vise encore la réalisation de compositions photochromes renfermant au moins un composé spiropyrannique répondant à la formule (I).

Les composés de formule I peuvent être obtenus sous une forme différente de celle indiquée. En particulier, ils peuvent correspondre à une forme dite ouverte répondant à la formule (II).

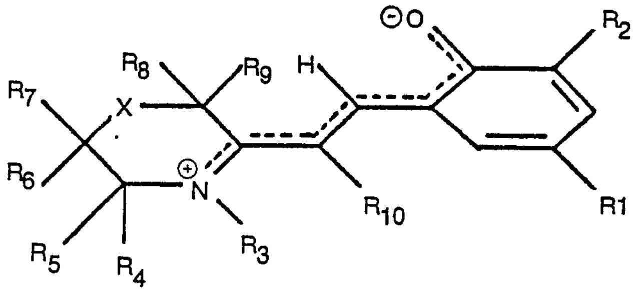

II

Sous l'action des photons, les composés de formule (I) se transforment en photomérocyanines colorées répondant à la formule (II) et qui par un traitement thermique se transformeront à nouveau en la forme fermée initiale.

Les produits selon l'invention sont particulièrement utiles à l'enregistrement d'informations par voie optique; ils peuvent être mis en oeuvre sur des supports transparents ou opaques pour l'enregistrement d'information stabilisées.

Pour préparer les composés selon l'invention, on opère de préférence en présence d'un solvant constitué par exemple par un alcool aliphatique ou encore par la benzène.

Pour obtenir les produits de l'invention sous forme déterminée ouverte ou bien fermée, il peut être nécessaire d'opérer en présence d'un déssèchant énergique tel que le sulfate de magnésium anhydre.

Pour rendre le milieu basique, on a recours de préférence à la pipéridine ou à une base azotée de force analogue.

Pour effectuer la condensation désirée, on porte le milieu réactionnel à une température comprise entre au moins 60°C et la température d'ébullition du solvant utilisé. De préférence, on opère à cette dernière température.

Pour isolen le spiropyranne, il est avantageux de faire une chromatographie sur colonne. L'absorbant peut être constitué par un gel de silice ou d'alumine de 0 à 10% d'eau, en particulier de 4 à 10%. Comme éluant on utilise un solvant organique aromatique tel que le benzène ou le toluène.

Bien entendu, la condensation peut être réalisée avec un aldéhyde de formule (III) comportant les substituants $R_1$ et $R_2$ tels que définis parmi lesquels les groupements classiques $NO_2$ et $OCH_3$ sont préférés.

Le sel quaternaire de formule (IV) mis en oeuvre dans l'étape de condensation est obtenu en faisant réagir un agent d'alkylation de formule (V) sur une base de formule (VI).

Cette réaction de quaternisation peut être effectuée avec ou sans solvant, de préférence à une température comprise entre environ la température ambiante et la température d'ébullition de l'agent d'alkylation.

Lorsqu'on opère dans un solvant, la température est comprise entre la température ambiante et la température de reflux du solvant. Ce solvant peut être choisi parmi les éthers.

Les exemples suivants illustrent la préparation d'un certain nombre de composés conformes à l'invention et qui se déroule en trois étapes de synthèses:

a) préparation des tétrahydro-3,4,5,6 pyridine et *2H*-dihydro-5,6 thiazine-1,4.

b) réaction de quaternisation avec un agent d'alkylation,

c) condensation du sel quaternaire ainsi obtenu avec un aldéhyde orthohydroxylé.

I — Composés spiropyranniques dans lesquels $X = CH_2$.

Exemple 1

Synthèse du spiropyranne de formule (XI).

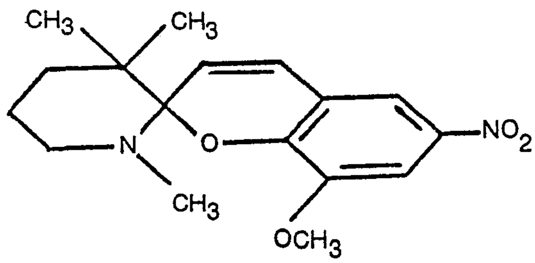

(XI)

a) A 15 g de chloro-5 diméthyl-2,2 pentanenitrile, dilué dans 60 ml d'éther anhydre, on ajoute goutte à goutte, 0,1 mole de méthyllithium en solution dans 200 ml d'éther anhydre, sous atmosphère inerte (azote ou argon). Le milieu réactionnel est maintenu entre 0° et 5°C pendant l'addition et ensuite pendant 30 mn, puis à température ambiante pendant 4 heures. Après hydrolyse, le triméthyl-2,3,3 tétrahydro-3,4,5,6 pyridine est extraite à l'éther. Ce composé est ensuite isolé par distillation. On obtient un liquide incolore P $éb_{14}$ = 60°C; le rendement en Δ 1-pipéridéine est de 76%.

b) A la Δ 1-pipéridéine précédemment obtenue on ajoute un excés d'iodure de méthyle; à température ordinaire la réaction est assez violente. Après 8 heures de repos, le sel formé est lavé à l'éther anhydre. One obtient des cristaux jaunâtres hygroscopiques; le rendement est quantitatif.

c) 5,34 g ($2.10^{-2}$ moles) du sel précédent et 3,94 g ($2.10^{-2}$ moles) de méthoxy-3 nitro-5 salicylaldéhyde sont mis en solution dans 100 $cm^3$ de benzène anhydre. On ajoute 2 $cm^3$ de pipéridine et porte au reflux pendant une heure trente la solution rouge foncée résultante. La solution benzénique est chromatographiée sur alumine à 10% d'eau (éluant: benzène); on retire de la fraction de tête un solide que l'on purifie par recristallisation dans l'éthanol. On obtient des cristaux jaunes dont le point de fusion est de 127°C avec un rendement de 60%.

Exemple 2

Synthèse de spiropyranne de formule (XII).

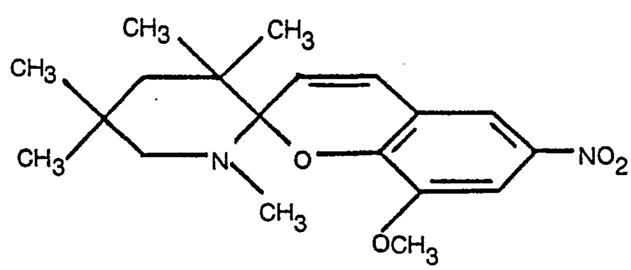

(XII)

a) A 22 g de bromo-5 tétraméthyl-2,2,4,4 pentane nitrile dans 60 ml d'éther anhydre, on ajoute goutte à goutte, 0,1 mole de méthyllithium en solution dans 200 ml d'éther anhydre, sous atmosphère inerte. Le milieu réactionnel est maintenu entre 0 et 5°C pendant l'addition et ensuite pendant 30 mn, puis à température ambiante pendant 4 heures. Après hydrolyse, on extrait la phase aqueuse à l'éther. La pentaméthyl-2,3,3,5,5 tétrahydro-3,4,5,6 pyridine est ensuite isolée par distillation. On obtient un liquide incolore de P $éb_{10}$—2 = 43°C avec un rendement de 70% en Δ1-pipéridéine.

b) A la Δ1-pipéridéine obtenue ci-dessus, on ajoute un excès d'iodure de méthyle. Après avoir laissé reposer une nuit, on ajoute une petite quantité d'éther anhydre au milieu réactionnel et on précipite ainsi les cristaux de sel. Ils sont légérement colorés en brun et hygroscopiques; le rendement est quantitatif.

c) 2,95 g ($10^{-2}$ moles) du sel précédent et 1,97 g ($10^{-2}$ moles) de méthoxy-3 nitro-5 salicylaldéhyde sont mis en solution dans 100 $cm^3$ de benzène anhydre. On ajoute 1 $cm^3$ de pipéridine et porte au reflux pendant une heure trente. La solution rouge foncée est chromatographiée sur alumine à 10% d'eau (éluant: benzène); un retire de la fraction de tête un solide qu'on purifie par recristallisation dans l'éthanol. On obtient des cristaux jaunes de P.F. = 102°C avec un rendement de 45%.

Exemple 3

Synthèse du spiropyranne de formule (XIII)

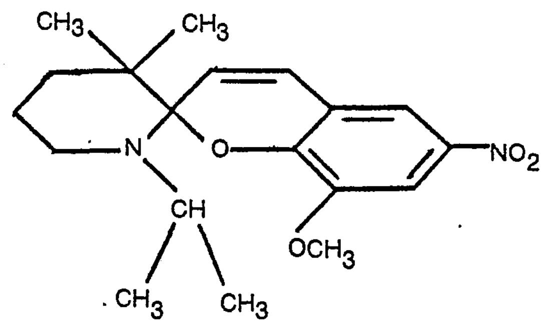

(XIII)

a) A 15 g de chloro-5 diméthyl-2,2 pentane nitrile, dilué dans 60 ml d'éther anhydre, on ajoute goutte à goutte 0,1 mole de méthyl-lithium en solution dans 200 ml d'éther anhydre, sous atmosphère inerte (argon ou azote). Le milieu réactionnel est maintenu entre 0° et 5°C, pendant l'addition et ensuite pendant 30 mn, puis à température ambiante pendant 4 heures. Après hydrolyse, on extrait à l'éther. La triméthyl-2,3,3 tétrahydro-3,4,5,6 pyridine est ensuite isolée par distillation. On obtient un liquide incolore de P $éb_{14} = 60°C$ avec un rendement de 76% en Δ1-pipéridéine.

b) A la Δ1-pipéridéine ainsi obtenue, on ajoute un excès d'iodure d'isopropyle et on porte un reflux pendant 4 heures. Puis on laisse reposer à la température ambiante; on ajoute ensuite un peu d'éther séché sur sodium et on isole les cristaux jaunâtres formés que l'on conserve ensuite dans un dessicateur sur anhydride phosphorique. Le rendement est quantitatif.

c) 2,95 g ($10^{-2}$ moles) du sel précédent et 1,97 g ($10^{-2}$ moles) de méthoxy-3 nitro-5 salicylaldéhyde sont mis en solution dans 100 $cm^3$ de benzène anhydre. On ajoute 1 $cm^3$ de pipéridine et on porte au reflux pendant une heure trente. La solution benzénique résultante est chromatographiée sur alumine à 10% d'eau (éluant: benzène). La fraction de tête donne un solide qu'on purifie par recristallisation dans l'éthanol. On obtient des cristaux de P.F. : 123°C avec un rendement de 55%.

Exemple 4

Synthèse du spiropyranne de formule (XIV)

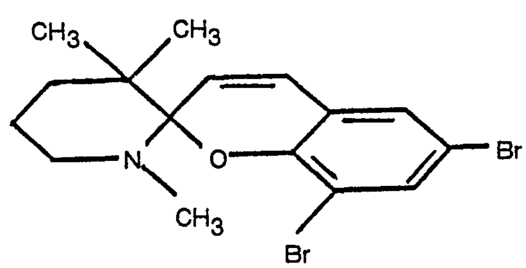

(XIV)

a) A 15 g de chloro-5 diméthyl-2,2 pentane nitrile, dilué dans 60 ml d'éther anhydre, on ajoute, goutte à goutte 0,1 mole de méthyllithium en solution dans 200 ml d'éther anhydre, sous atmosphère inerte. Le milieu réactionnel est maintenu entre 0°C et 5°C pendant l'addition et ensuite pendant 30 mn, puis à température ambiante pendant 4 heures. Après hydrolyse la triméthyl-2,3,3 tétrahydro-3,4,5,6 pyridine est extraite à l'éther. Ce composé est ensuite isolé par distillation. On obtient un liquide incolore de P $éb_{14} = 60°C$ avec un rendement de 76% en Δ1-pipéridéine.

b) A la Δ1-pipéridéine ainsi obtenue, on ajoute un excès d'iodure de méthyle; à température ambiante la réaction est assez violente. Après 8 heures de repos, le sel formé est lavé à l'éther anhydre. On obtient des cristaux jaunâtres; le rendement est quantitatif.

c) 5,34 g ($2.10^{-2}$ moles) du sel précédent et 5,60 g ($2.10^{-2}$ moles) du dibromo-3,5 salicylaldéhyde sont mis en solution dans 100 $cm^3$ de benzène anhydre. On ajoute 2 $cm^3$ de pipéridine et porte à reflux une heure trente. La solution benzénique est chromatographiée sur alumine à 10% d'eau (éluant: benzène); on retire de la fraction de tête un solide que l'on purifie par recristallisation dans un mélange d'éthanolbenzène. On obtient des cristaux blancs de P.F. = 128°C avec un rendement de 50%.

Exemple 5

Synthèse du spiropyranne de formule (XV)

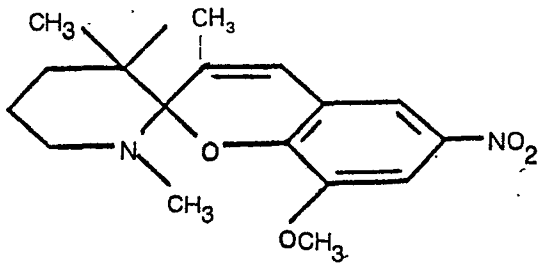

(XV)

a) A 15 g de chloro-5 diméthyl 2,2 pentanenitrile dilué dans 60 ml d'éther anhydre, on ajoute goutte à goutte, 0,1 mole d'éthyllithium en solution dans 200 ml d'éther anhydre, sous atmosphère inerte (azote ou argon). Le milieu réactionnel est maintenu entre 0° et 5°C pendant l'addition, et ensuite pendant 30 mn, puis à température ambiante pendant 4 heures. Après hydrolyse la diméthyl-3,3 éthyl-2 tétrahydro-3,4,5,6 pyridine est extraite à l'éther. Ce composé est ensuite isolé par distillation. On obtient un liquide incolore P $éb_{14} = 66$°C le rendement en Δ1-pipéridéine est de 65%.

b) A la Δ1-pipéridéine précédemment obtenue on ajoute un excès d'iodure de méthyle; à température ordinaire la réaction est assez violente. Après 8 heures de repos, le sel formé est lavé à l'éther anhydre. On obtient des cristaux hygroscopiques; le rendement est quantitatif.

c) 5,62 g ($2.10^{-2}$ moles) du sel précédent et 3,94 g ($2.10^{-2}$ moles) de méthoxy-3 nitro-5 silicylaldéhyde sont mises en solution dans 100 $cm^3$ de benzène anhydre. On ajoute 2 $cm^3$ de pipéridine et porte au reflux pendant une heure trente la solution rouge foncée résultante. La solution benzénique est chromatographiée sur alumine à 10% d'eau (éluant — benzène); on retire de la fraction de tête un solide que l'on purifie par recristallisation dans l'éthanol. On obtient des cristaux jaunes de PF = 156°C avec un rendement de 10%.

Exemple 6

Synthèse du spiropyranne de formule (XVI)

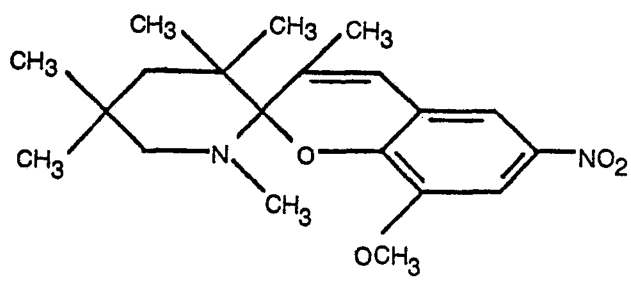

(XVI)

a) A 22 g de bromo-5 tétraméthyl-2,2,4,4 pentane nitrile dans 60 ml d'éther anhydre, on ajoute goutte à goutte, 0,1 mole d'éthyllithium en solution dans 200 ml d'éther anhydre, sous atmosphère inerte. Ce milieu réactionnel est maintenu entre 0° et 5°C pendant l'addition et ensuite pendant 30 mn, puis à température ambiante pendant 4 heures. Après hydrolyse, on extrait la phase aqueuse à l'éther. La tétraméthyl 3,3,5,5 éthyl-2 tétrahydro-3,4,5,6 pyridine est ensuite isolée par distillation. On obtient un liquide incolore de P $éb_{2.10^{-2}} = 40$°C avec un rendement de 40%.

b) A la Δ1-pipéridéine obtenue ci-dessus, on ajoute un excès d'iodure de méthyle. Après avoir laissé reposer une nuit, on ajoute une petite quantité d'éther anhydre au milieu réactionnel et on précipite ainsi les cristaux de sel. Ils sont légèrement colorés en brun et hygroscopiques; le rendement est quantitatif.

c) 3,09 g ($10^{-2}$ moles) du sel précédent et 1,97 g ($10^{-2}$ moles) de méthoxy-3 nitro-5 salicylaldéhyde sont mis en solution dans 100 $cm^3$ de benzène anhydre. On ajoute 1 $cm^3$ de pipéridine et porte à reflux pendant une heure trente. La solution rouge foncée est chromatographiée sur alumine à 10% d'eau (éluant = benzène) on retire de la fraction de tête un solide qu'on purifie par recristallisation dans l'éthanol. On obtient des cristaux jaunes de PF = 122°C avec un rendement de 14%.

Tableau 1

Spiropyrannes de formule générale Ia.

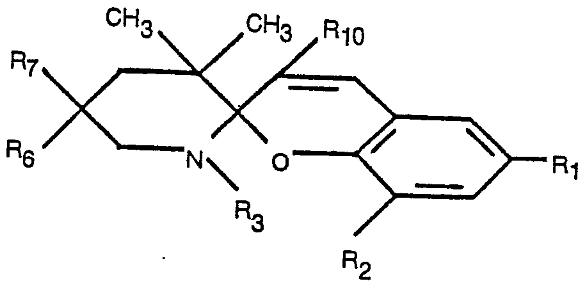

(Ia)

En opérant conformément aux exemples 1 à 6, on obtient les composés spiropyranniques de structures Ia ci-dessus, dans lesquels X représente un groupement $CH_2$, $R_4$ et $R_5$ des hydrogènes, $R_8$ et $R_9$ des groupements méthyle. Les exemples 7 à 17 sont rassemblés dans le tableau 1; les groupements $R_1$, $R_2$, $R_3$, $R_6$, $R_7$ et $R_{10}$ présents dans les composés de formule I ont la signification mentionnée.

| Exemples | $R_1$ | $R_2$ | $R_3$ | $R_6$ | $R_7$ | $R_{10}$ | P.F. (°C) | Redt (%) |
|---|---|---|---|---|---|---|---|---|
| 7 | $NO_2$ | $OCH_3$ | $CH_3$ | $CH_3$ | H | H | 125 | 60 |
| 8 | $NO_2$ | $OCH_3$ | $C_2H_5$ | H | H | H | 114 | 60 |
| 9 | $NO_2$ | $OCH_3$ | $CH_2C_6H_5$ | H | H | H | 141 | 35 |
| 10 | $OCH_3$ | $NO_2$ | $CH_3$ | H | H | H | 68 | 62 |
| 11 | I | I | $CH_3$ | H | H | H | 83 | 50 |
| 12 | $-C(=O)-C_6H_5$ | H | $CH_3$ | H | H | H | non cristallisé | 55 |
| 13 | $NO_2$ | $NO_2$ | $CH_3$ | H | H | H | 137 | 12,5 |
| 14 | $NO_2$ | H | $CH_3$ | H | H | H | 117 | 22 |
| 15 | H | H | $CH_3$ | H | H | H | liquide visqueux | 28 |
| 16 | $NO_2$ | $OCH_3$ | $CH_3$ | H | $CH_3$ | $CH_3$ | 100 | 5 |
| 17 | $NO_2$ | $OCH_3$ | $CH_3$ | H | H | $C_2H_5$ | 127 | 8 |

II – Compose spiropyranniques dans lesquels X = S ($R_{10}$ = H)

Exemple 18

Synthèse du spiropyranne de formule (XVII)

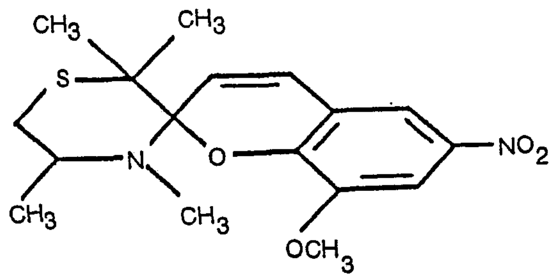

(XVII)

a) Dans un ballon, on chauffe à 40°C au bain-marie 33 g (0,58 mole) de méthyl-2 aziridine et 1 pastille de soude, sous atmosphère d'azote. Puis on ajoute goutte à goutte 59 g (0,5 mole) de mercapto-3 méthyl-3 butanone.

Après la fin de l'addition, le mélange est chauffé pendant 1 heure à 60°C. Après lavage et extraction, on isole par distillation la tétraméthyl-2, 2,3,5 *2H*-dihydro-5,6 thiazine-1,4. On obtient un liquide de P $éb_{5.10}$—2 = 37°C avec un rendement de 95%.

b) A 3,14 g de thiazine-1,4 on ajoute un excès d'iodure de méthyle. On laisse la quaternisation se faire pendant une nuit, à température ambiante. On obtient des cristaux blancs après lavage à l'éther anhydre. Le rendement est sensiblement quantitatif.

c) 5,98 g ($2.10^{-2}$ moles) du sel précédent et 3,94 g ($2.10^{-2}$ moles) de méthoxy-3 nitro-5 salicylaldéhyde sont mis en solution dans du benzène anhydre. On ajoute 2 $cm^3$ de pipéridine et porte au reflux pendant 1 heure; pendant le reflux le benzène est séché sur du sulfate de magnésium. La solution benzénique est chromatographiée sur alumine à 10% (éluant: benzène). La fraction jaune de tête est évaporée et on isole un solide, que l'on cristallise dans l'éthanol. On obtient des cristaux jaunes de P.F. = 126°C avec un rendement de 36%.

Exemple 19

Synthèse du spiropyranne de formule (XVIII)

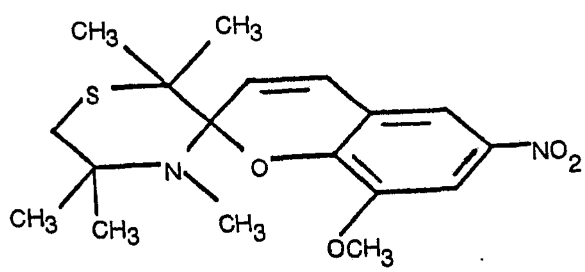

(XVIII)

a) Dans un ballon, on chauffe à 40°C au bain-marie, en agitant 20,6 g (0,29 mole) de diméthyl-2,2 aziridine et 1 pastille de soude, sous atmosphère d'azote. Puis on ajoute goutte à goutte 29,5 g (0,25 mole) de mercapto-3 méthyl-3 butanone. Le mélange est ensuite chauffé à 60°C pendant 1 heure. Après lavage et extraction, on distille la pentaméthyl-2,2,3,5,5 2H-dihydro-5,6 thiazine-1,4. On obtient un liquide incolore de P $éb_{0,6}$ = 45°C avec un rendement de 68%.

b) A la thiazine ainsi obtenue on ajoute un excès d'iodure de méthyle. On laisse reposer à température ambiante pendant 2 à 3 jours. On obtient des cristaux blancs avec un rendement quantitatif.

c) 6,2 g ($2.10^{-2}$ moles) du sel précédent et 3,94 g de méthoxy-3 nitro-5 salicylaldéhyde sont dissouts dans 100 $cm^3$ de benzène anhydre. 2 $cm^3$ de pipéridine sont ajoutés et le mélange réactionnel est porté à reflux, pendant 1 heure trente; le benzène est désséché au cours de la réaction sur du sulfate de magnésium. La solution benzénique est chromatographiée sur alumine à 10% d'eau (éluant: benzène). De la fraction jaune pâle on retire un solide que l'on purifie par recristallisation dans l'éthanol. On obtient des cristaux jaunes de P.F. = 168°C avec un rendement de 29%.

Exemple 20

Synthèse du spiropyranne de formule (XIX)

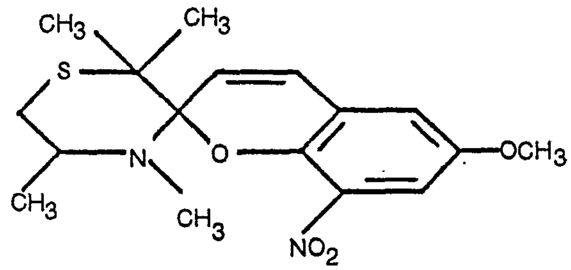

(XIX)

a) Dans un ballon, on chauffe à 40°C au bain-marie 33 g (0,58 mole) de méthyl-2 aziridine et 1 pastille de soude, sous atmosphère d'azote. Puis on ajoute goutte à goutte 59 g (0,5 mole) de mercapto-3 méthyl-3 butanone. A la fine de l'addition, on chauffe le mélange à 60°C pendant 1 heure. Après lavage et extraction, on isole par distillation la tétraméthyl-2,2,3,5 2H-dihydro-5,6 thiazine-1,4. On obtient un liquide de P $éb_{5.10}$–2 = 37°C avec un rendement de 95%.

b) A la thiazine-1,4 ainsi obtenue, on ajoute un excès d'iodure de méthyle. La quaternisation se fait pendant une nuit à température ambiante. Des cristaux blancs sont obtenus après lavage à l'éther anhydre. Le rendement est sensiblement quantitatif.

c) 5,98 g ($2.10^{-2}$ moles) de sel quaternaire et 3,94 g ($2.10^{-2}$ mole) de méthoxy-5 nitro-3 salicylaldéhyde sont mis en solution dans 100 $cm^3$ de benzène anhydre. On ajoute 2 $cm^3$ de pipéridine et on porte à reflux pendant 20 mn. La solution benzénique est chromatographiée sur alumine à 10% d'eau. On évapore la fraction de tête et isole un solide que l'on purifie par recristallisation dans l'éthanol. On obtient des cristaux jaunes citron de P.F. = 116°C avec un rendement de 48%.

Exemple 21

Synthèse du spiropyranne de formule (XX)

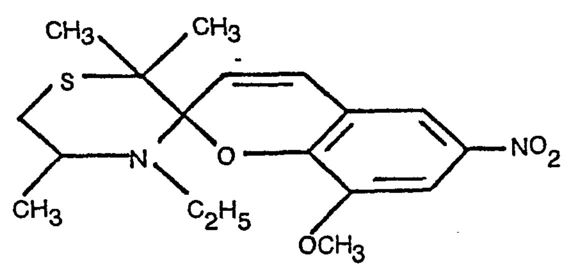

(XX)

a) Dans un ballon, on chauffe au bain-marie à 40°C, 33 g (0,58 mole) de méthyl-2 aziridine et 1 pastille de soude, sous atmosphère inerte. Puis on ajoute goutte à goutte 59 g (0,5 mole) de mercapto-3 méthyl-3 butanone. A la fin de l'addition, on chauffe le mélange à 60°C pendant 1 heure. Après lavage et extraction, on distille la tétraméthyl-2,2,3,5 *2H*-dihydro-5,6 thiazine-1,4 liquide P $éb_{5.10}$–2 = 37°C; rendement 95%.

b) A la thiazine-1,4 ainsi obtenue, on ajoute un excès d'iodure d'éthyle et on porte au reflux pendant 4 heures. On laisse ensuite reposer à température ambiante. Après lavage à l'acétone, on obtient des cristaux blancs hygroscopiques. Le rendement est sensiblement quantitatif.

c) 6,26 g ($2.10^{-2}$ moles) due sel précédent et 3,94 g de méthoxy-3 nitro-5 salicylaldéhyde sont mis en solution dans 100 $cm^3$ de benzène anhydre. On ajoute 2 $cm^3$ de pipéridine et porte au reflux pendant 15 mn, le benzène est séché sur sulfate de magnésium au cours de la réaction. La solution benzénique est chromatographiée sur alumine à 10% d'eau (éluant: benzène). La fraction rouge orangée de tête est évaporée; on isole le spiropyranne sous forme de laque brune que l'on obtient avec un rendement de 58%.

En opérant de la manière enseignée des les exemples 18 à 21 on prépare des composés spiropyranniques répondant à la formule (Ib) ci-dessous dans laquelle $R_6 = R_7 = H$, $R_8 = R_9 = CH_3$; les groupements $R_1$ à $R_5$ ont la signification mentionnée ci-après.

TABLEAU 2 : Spiropyrannes de formule generale (Ib)

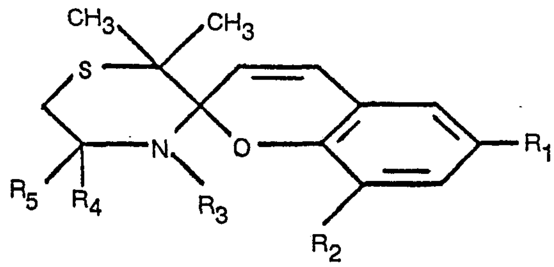

(Ib)

| Exemples | $R_1$ | $R_2$ | $R_3$ | $R_4$ | $R_5$ | P.F. (°C) | Rendt % |
|---|---|---|---|---|---|---|---|
| 22 | I | I | $CH_3$ | $CH_3$ | H | 132 | 42 |
| 23 | Br | Br | $CH_3$ | $CH_3$ | H | 119 | 72 |
| 24 | $CO-C_6H_5$ | H | $CH_3$ | $CH_3$ | H | non cristallise | 51 |
| 25 | $NO_2$ | $NO_2$ | $CH_3$ | $CH_3$ | H | 119 | 1 |
| 26 | $NO_2$ | $OCH_3$ | $iC_3H_7$ | $CH_3$ | H | laque | 44 |

Comme on l'a indiqué précédemment, les dérivés selon l'invention sont doués de propriétés photochromiques.

Pour étudier ces propriétés, on a utilisé la technique de photolyse éclair et mesuré la variation d'absorbance du maximum d'absorption de la forme colorée et la constante cinétique de décoloration thermique de solutions spiropyranniques dans du toluène anhydre (20 ppm d'eau). Ces mesures sont effectuées 1 25°C dans des cuves de 10 cm de longueur et de 3 cm d'épaisseur, et avec une énergie de photolyse constante de 800 joules (avec un spectre étalé). Le solvant utilisé est le toluène et la concentration de l'échantillon est de $5.10^{-5}$ moles/l.

Deux enregistrements photographiques permettent de déterminer les caractéristiques spectrales et cinétiques au cours de la décoloration de l'échantillon photochrome.

On rapporte dans le tableau 3 ci-après les résultats obtenus avec un certain nombre de composés selon l'invention. Dans ce tableau, on indique les valeurs de l'absorbance initiale $A_n$ au moment de la photolyse la constante de décoloration thermique $K\Delta$ en $S^{-1}$ ainsi que la valeur de $\lambda$ de la forme ouverte, mesurées pour les composés obtenus selon les exemples 1 à 20, 22 à 25.

TABLEAU 3

| Exemples | λ (nm) F.O. | Ao | K Δen $s^{-1}$ (25°C) |
|---|---|---|---|
| 1 | 580 | 1,42 | 0,42 |
| 2 | 580 | 1,68 | 0,36 |
| 3 | 530 | 0,66 | 0,17 |
| 4 | 580 | 0,05 | 5,6 |
| 5 | 430 | 0,125 | 0,46 |
| 6 | 428 | 0,175 | 0,32 |
| 7 | 580 | 1,68 | 0,35 |
| 8 | 530 | 1,18 | 0,15 |
| 9 | 530 | 0,73 | 0,60 |
| 10 | 600 | 0,07 | 23 |
| 11 | 580 | 0,03 | 8,5 |
| 12 | 580 | 1,24 | 0,73 |
| 13 | 530 | 0,27 | 0,014 |
| 14 | 580 | 0,30 | 0,02 |
| 15 | 560 | 0,02 | 59 |
| 16 | 436 | 0,57 | 0,15 |
| 17 | 420 | 0,172 | 0,23 |
| 18 | 600 | 1,22 | 75 |
| 19 | 590 | 1,40 | 50 |
| 20 | 620 | 0,09 | 171 |
| 22 | 600 | 0,08 | 147 |
| 23 | 600 | 0,03 | 106 |
| 24 | 600 | 0,24 | 160 |
| 25 | 560 | 0,76 | 19,5 |

Dans le tableau 4, on a rapporté les valeurs obtenues dans les mêmes conditions opératoires pour un certain nombre de composés indoliniques de l'art antérieur décrit dans le brevet français 2 008 056 et répondant à la formule générale suivante:

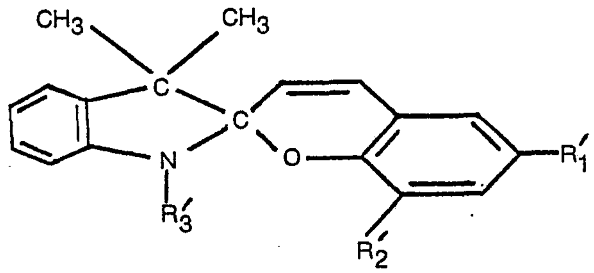

TABLEAU 4

| $R_1'$ | $R_2'$ | $R_3'$ | λ (nm) F.O. | Ao | kΔ ($S^{-1}$) |
|---|---|---|---|---|---|
| $NO_2$ | $OCH_3$ | $CH_3$ | 620 | 1,0 | $2,3.10^{-2}$ |
| $OCH_3$ | $NO_2$ | $CH_3$ | 640 | 0,30 | $8,9.10^{-1}$ |
| $NO_2$ | H | $CH_3$ | 620 | 0,78 | $2,3.10^{-1}$ |
| $CO{-}C_6H_5$ | H | $C_6H_5$ | 620 | 0,72 | 2,32 |
| I | I | $CH_3$ | 620 | 0,12 | 0,11 |
| Br | Br | $CH_3$ | 620 | 0,05 | 0,143 |
| H | H | $CH_3$ | 580 | – | 0,95 |

Dans le tableau 5, on a rapporté les valeurs obtenues dans les mêmes conditions opératoires pour un certain nombre de composés benzothiazoliniques connus décrits dans la thèse de M. GUGLIELMETTI, Marseille 1967 répondant à la formule générale suivante:

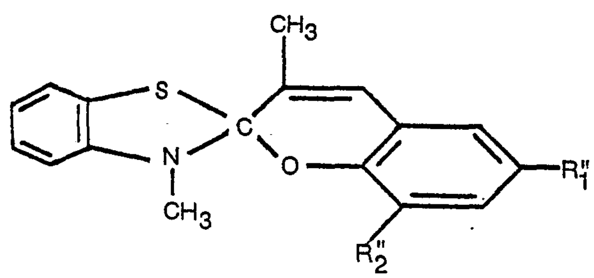

TABLEAU 5

| $R''_1$ | $R''_2$ | λ (nm) F.O. | Ao | KΔ ($s^{-1}$) |
|---|---|---|---|---|
| $NO_2$ | $OCH_3$ | 620 | 0,25 | 2,6 |
| $OCH_3$ | $NO_2$ | 645 | 0,04 | 30 35 |
| $NO_2$ | H | 580 | 0,06 | 1,3 |
| $COC_6H_5$ | H | – | per coloré | 0,136 |

On a également effectué les mêmes mesures relativement à un composé thiazinique-1.3 connu décrit dans la revue Heterocyclic Chemistry vol 15, 1439—46 (1978) de formule:

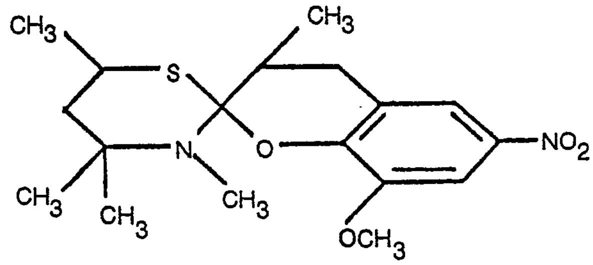

$\lambda$ (F.O.) : 420 nm : 620 nm :
Ao : 0,29 : faible :
K = 138s$^{-1}$ : 0,05 :

La comparaison des résultats obtenus pour les composés selon l'invention et pour les composés de l'art antérieur montre la remarquable aptitude des premiers à se colorer en accroissant leur plage d'utilisation aussi bien au niveau cinétique que spectroscopique.

Il convient de mentionner les propriétés spectroscopiques tout à fait remarquables des composés pipéridiniques dans lesquels $R_{10}$ est représenté par un groupement méthyle ou éthyle. Ces composés présentent une unique bande d'absorption vers 420 nm, c'est à dire qu'ils absorbent dans le bleu et se colorent en jaune sous l'action photonique. Ainsi, les composés selon l'invention permettent d'accéder à des pellicules photochromes à trois couleurs (jaune bleu et rouge).

Il est intéressant de remarquer également que les constantes cinétiques de décoloration thermique diffèrent selon la nature des substituants des composés selon l'invention, ce qui traduit une stabilité plus au moins grande des formes ouvertes, ou photomérocyanines, des dérivés étudiés.

On constate de façon générale que le dérivés selon l'invention dans lesquels X est un groupement $CH_2$, $R_1$=$NO_2$ et $R_2$=$OCH_3$ conduisent à des photomérocyanines qui sont grandement stabilisées. De ce fait, elles sont particulièrement intéressantes pour less applications dans lesquelles on désire stocker une information et sont utilisées avantageusement pour l'enregistrement d'informations stabilisées sur des supports transparents, par exemple des pellicules, ou opaques tels que du papier. Ainsi, ces dérivés sont particulièrement appropriés notamment à la reproduction de documents (procédé de reprographie auto-processeurs), à la photographie non conventionnelle, à l'obtention d'images latentes stables, effaçables thermiquement. En particulier, ces dérivés sont avantageusement utilisés pour la réalisation de pellicules photochromes et de papiers photochromiques. Les pellicules peuvent être obtenues à partir de préparations comprenant de l'acétobutyrate de cellulose ou tout autre support en matière plastique.

Pour réaliser les papiers photochromiques, on étend sur du papier une préparation comportant de 1 à 5% en poids de dérivé selon l'invention, de préférence de l'ordre de 2%. Une telle préparation renferme avantageusement de 20 à 80 g, de préférence 40 à 60 g de substance semiconductrice telle que $TiO_2$ ou ZnO, de 5 à 25 g, de préférence de 10 à 20 g d'un liant tel que celui constitué par une résine commercialisée sous la dénomination RP 1022, de 10 à 40 g, de préférence de 20 à 30 g d'un solvant organique tel que le toluène, de 2 à 30 g, de préférence de 5 à 15 g d'un alcool tel que l'éthanol et de 0,5 à 5 g, de préférence 1 à 3 g de spiropyranne.

Une préparation préférée utilisée conformément à l'invention renferme environ 50 g de ladite substance semi-conductrice, environ 15 g du susdit liant, environ 25 g de solvant organique, environ 10 g d'alcool et environ 2 g de spiropyranne.

Par irradiation du papier photochromique, on obtient une image contrastée et bien résolue qui est stabilisée quelque temps. Ces pellicules et papiers photochromiques ont de nombreuses applications notamment dans le domaine de la photographie artisanale, pour évaluer des négatifs, pour l'enregistrement des données affichées sur tube cathodique.

L'examen du tableau 3 montre également que les dérivés selon l'invention dans lesquels X est un atome de soufre, $R_1$=$NO_2$ et $R_2$=$OCH_3$, tout en ayant une bonne aptitude à se colorer, sont dotés de vitesses de décoloration thermique plus rapides.

Ainsi ces propriétés permettent d'utiliser les dérivés de ce type dans des applications où la vitesse d'effacement de l'information est un facteur déterminant. Tel est le cas, par exemple, des écrans de visualisation, du contrôle par la lumière de la transparence d'un diaphragme, du traitement des images dans les systèmes corrélateurs, des inscriptions d'images avec laser ultra-violet.

Revendications

1. Composés spiropyranniques, caractérisés en ce qu'ils répondent à la formule générale (I).

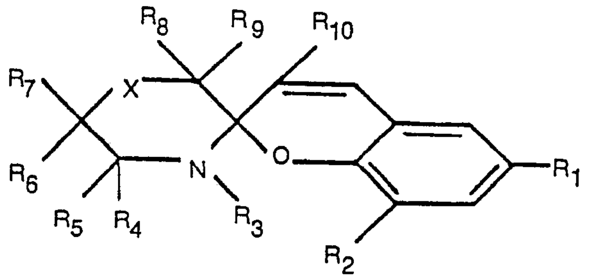

(I)

dans laquelle:

— X représente un groupement $CH_2$ ou un atome de soufre,

— $R_1$ et $R_2$ représentent des groupements accepteurs et/ou donneurs d'électrons,

— $R_3$ est un groupement alkyle, linéaire ou ramifié, comportant de 1 à 18 atomes de carbone, un groupement phénylalkyle dans lequel le groupe alkyle linéaire ou ramifié comporte de 1 à 5 atomes de carbone, un groupement alcényle comportant 2 à 10 atomes de carbone;

— $R_4$, $R_5$, $R_6$, $R_7$, $R_8$ et $R_9$ présentent l'une quelconque des significations indiquées pour $R_3$, représentent un atome d'hydrogène, ou participent deux à deux à la formation d'un homocycle saturé de 5 à 10 atomes de carbone, soit avec le groupement se trouvant sur le même atome de carbone, soit avec le groupement en ortho;

— $R_{10}$ représente si $X = S$ un atome d'hydrogène et si $X = CH_2$ un atome d'hydrogène, un groupement alkyle comportant 1 à 18 atomes de carbone, un groupement alcényle comportant 2 à 10 atomes de carbone.

2. Composés selon la revendication 1, caractérisés en ce que:

— $R_1$ et $R_2$ sont choisis dans le groupe constitué par l'hydrogène, $NO_2$, —CN, les halogènes, les substituants $—COC_6H_5$, méthoxy, les groupements alkyle, mercaptoalkyle, les éthers de formule $—CH_2OR$, des thioéthers de formule $CH_2$ SR et des amines de formule $—CH_2NR_2$ dans lesquelles R représente un groupe alkyle,

— $R_3$ est choisi parmi les substituants méthyle, éthyle, isopropyle, n-butyle, benzyle ou phénéthyle;

— $R_4$, $R_5$, $R_6$, $R_7$, $R_8$ et $R_9$ présentent l'une des significations indiquées pour $R_3$, ou représentent deux à deux, avec le groupement en ortho, un groupe

— $(CH_2)$ n- dans lequel n est un nombre comprise entre 5 et 10 ou un atome d'hydrogène;

— $R_{10}$ représente si $X = S$ un atome d'hydrogène et si $X = CH_2$ un atome d'hydrogène, un substituant méthyle ou éthyle.

3. Composés selon la revendication 2, caractérisés en ce que:

— $R_1$ est représenté par $NO_2$, $OCH_3$, $—COC_6$ $H_5$, I ou Br;

— $R_2$ est représenté par $OCH_3$, $NO_2$, H, I ou Br;

— $R_4$ à $R_9$ sont représentés par un atome d'hydrogène ou un groupement méthyle, éthyle, isopropyle, n-butyle, benzyle ou phénétyle.

4. Composés selon la revendication 3, caractérisée en ce que $R_3$ est représenté par un groupement méthyle, éthyle ou isopropyle et $R_4$ à $R_9$ sont choisis dans le groupe constitué par l'hydrogène et les groupements méthyle, éthyle ou isopropyle.

5. Composés selon l'une quelconque des revendications 2 à 4, caractérisés par le fait que X représente un groupement $CH_2$, $R_4$ et $R_5$ des atomes d'hydrogène, et $R_8$ et $R_9$ des substituants méthyle et $R_{10}$ un groupement méthyle ou éthyle.

6. Composés selon l'une quelconque des revendications 2 à 4, caractérisé par le fait que X représente un atome de soufre, $R_6$ et $R_7$ des atomes d'hydrogène et $R_8$ et $R_9$ des substituants méthyle et $R_{10}$ un atome d'hydrogène.

7. Procédé de préparation de dérivés spiropyranniques selon l'une des revendications 1 à 6, caractérisé par le fait qu'il comprend la condensation d'un aldéhyde aromatique orthohydroxylé de formule (III):

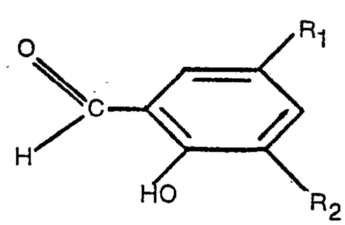

(III)

avec un sel quartenaire de formule (IV):

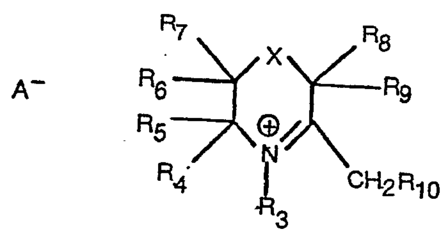

(IV)

dans lesquelles X et $R_1$ à $R_{10}$ ont les significations précédemment indiquées et A représente un anion choisi dans le groupe des anions iodure, tosylate, méthylsulfate.

8. Procédé selon la revendication 7, caractérisé par le fait que la condensation et réalisée en milieu basique.

9. Procédé selon la revendication 7 ou 8, caractérisé par le fait que le sel quartenaire de formule (IV), est obtenu en faisant réagir un agent d'alkylation de formule (V) sur une base de formule (VI) selon la réaction:

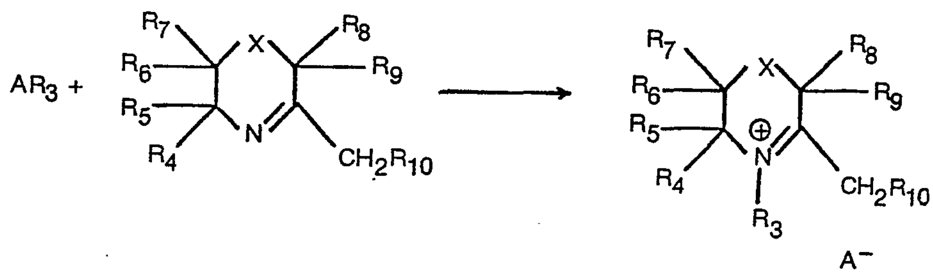

(V) (VI) (IV)

ou $R_3$ à $R_{10}$ X et A présentent les significations sus-indiquées.

10. Procédé selon la revendication 9, caractérisé par le fait que, pour obtenir les produits dans lesquels X représente un groupement $CH_2$, on prépare la Δ1-pipéridéine de formule (VI) par condensation de $LiCH_2R_{10}$ sur un nitrile ω-halogéné de formule VIII selon le schéma:

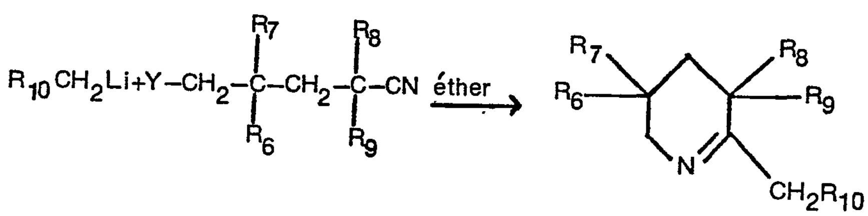


(VII) (VIII) (VI)

les groupements $R_6$ à $R_{10}$ présentent les significations indiquées et Y le chlore ou le brome.

11. Procédé selon la revendication 9, caractérisé un ce que pour obtenir les produits dans lesquels X représente un atome de soufre, on condense une mercaptocétone de formule (IX) sur une aziridine de formule (X), selon le schéma:

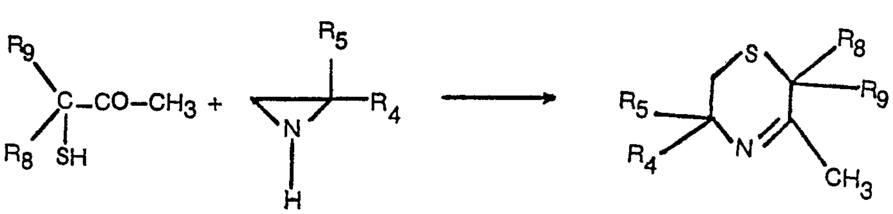

(IX) (X) (VI)

$R_4$, $R_5$, $R_8$ et $R_9$ ayant les significations sus-indiquées.

12. Composition photochrome, caractérisée en ce qu'elle comporte au moins un composé spiropyrannique selon l'une quelconque des revendications 1 à 6.

**Patentansprüche**

1. Spiropyranische Verbindungen, dadurch gekennzeichnet, dass sie der allgemeinen Formel entsprechen,

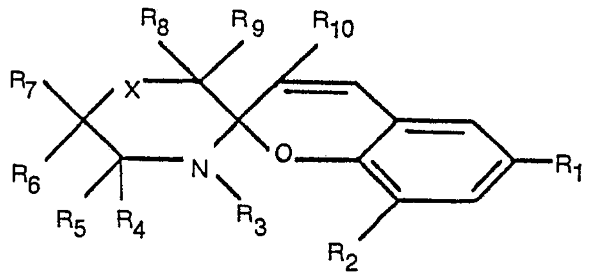

(I)

in der:

— X eine $CH_2$ gruppe oder ein Schwefel-Atom darstellt,

— $R_1$ und $R_2$ Gruppen darstellen, die Elektronen übernehmen und/oder abgeben,

— $R_3$ eine lineare oder verzweigte Alkyl-Gruppe mit 1 bis 18 C-Atomen, eine Phenylalkyl-Gruppe mit 1 bis 5 C-Atomen in der linearen oder verzweigten Alkyl-Gruppe oder eine Alkenylgruppe mit 2 bis 10 C-Atomen darstellen,

— $R_4$, $R_5$, $R_6$, $R_7$, $R_8$ und $R_9$ stellen eine der für $R_3$ angegebenen Konfigurationen dar und entsprechen einem H-Atom oder sind paarweise an der Bildung eines gesättigten Homozyklus mit 5 bis 10 C-Atomen beteiligt, und zwar entweder mit der Gruppe, die sich am gleichen C-Atom befindet, oder mit der Ortho-Gruppe,

— $R_{10}$ stellt, wenn X=S ein H-Atom und wenn X=$CH_2$ ein H-Atom ist, eine Alkylgruppe mit 1 bis 18 C-Atomen bzw.eine Alkenylgruppe mit 2 bis 10 C-Atomen dar.

2. Verbindungen nach Anspruch 1, dadurch gekennzeichnet, dass:

— $R_1$ und $R_2$ in der Gruppe gewählt werden, die besteht aus: Wasserstoff, $NO_2$, —CN, Halogenen, —$COC_6H_5$ und Methoxy-Ersatz, Alkylgruppen, Merkapto-Alkyl, Athern der Formel —$CH_2OR$, Thioäthern der Formel —$CH_2SR$ und Aminen der Formel —$CH_2NR_2$ in denen R eine Alkylgruppe darstellt,

— $R_3$ wird in den Ersatzstoffen Methyl, Athyl, Isopropyl, n-Butyl, Benzyl oder Phenäthyl gewählt.

— $R_4$, $R_5$, $R_6$, $R_7$, $R_8$ und $R_9$ stellen eine der für $R_3$ angegebenen Konfigurationen der oder entsprechen paarweise mit der Orthogruppe, einer —$(CH_2)_n$-Gruppe, in der n eine Zahl zwischen 5 und 10 oder ein H-Atome ist,

— $R_{10}$ stellt, wenn X=S eine H-Atom und wenn X=$CH_2$ ein H-Atom ist, einen Methyl-oder Äthyl-Ersatz dar.

3. Verbindungen nach Anspruch 2, dadurch gekennzeichnet dass:

— $R_1$ durch $NO_2$, $OCH_3$, —$COC_6H_5$, I oder Br dargestellt ist

— $R_2$ durch $OCH_3$, $NO_2$, H, I oder Br dargestellt ist

— $R_4$ bis $R_9$ durch ein H-Atome oder eine Methyl-, Athyl-, Isopropyl-, n-Butyl-, Benzyl- oder Phenetyl-Gruppe dargestellt sind.

4. Verbindungen nach Anspruch 3, dadurch gekennzeichnet dass $R_3$ durch eine Methyl-, Athyl- oder Isopropyl-Gruppe dargestellt ist und dass $R_4$ bis $R_9$ in der Gruppe gewählt werden, die aus H und den Methyl-, Athyl- oder Isopropyl-Gruppen besteht.

5. Verbindungen nach einem der Ansprüche 2 bis 4, dadurch gekennzeichnet, dass X eine $CH_2$-Gruppe und $R_4$ und $R_5$ H-Atome und $R_8$ und $R_9$ Methylersatz und $R_{10}$ eine Methyl- und Äthyl-Gruppe darstellen.

6. Verbindungen nach einem der Ansprüche 2 bis 4, dadurch gekennzeichnet, dass X ein S-Atom, $R_6$ und $R_7$ H-Atome und $R_6$ und $R_7$ Methylersatz und $R_{10}$ ein H-Atom darstellen.

7. Verfahren zur Herstellung von spiropyranischen Verbindungen nach einem der Ansprüche 1 bis 6, dadurch gekennzeichnet, dass es die Kondensierung eines aromatischen Orthohydroxyl-Aldehyds der Formel (III) und eines quaternären Salzes der Formel (IV) umfasst:

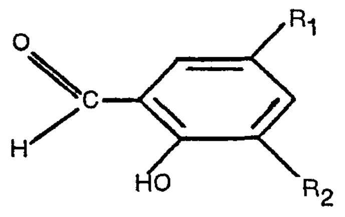 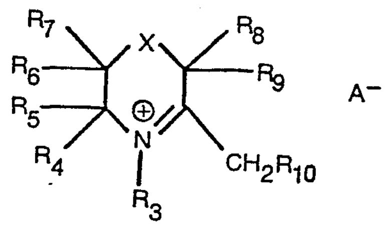

(III) (IV)

in der X und $R_1$ bis $R_{10}$ die oben angeführten Konfigurationnen und A ein Anion darstellen, das in der Gruppe der Anionen Jodid, Tosylat, Methylsulfat gewählt ist.

8. Verfahren nach Anspruch 7, dadurch gekennzeichnet, dass die Kondensation in basischer Umgebung erfolgt.

9. Verfahren nach Anspruch 7 oder 8, dadurch gekennzeichnet dass das quaternäre Salz der Formel (IV) dadurch erzeugt wird, dass man ein Alkyliermittel der Formel (V) auf der Basis der Formel (VI) nach folgender Reaktion reagieren lässt:

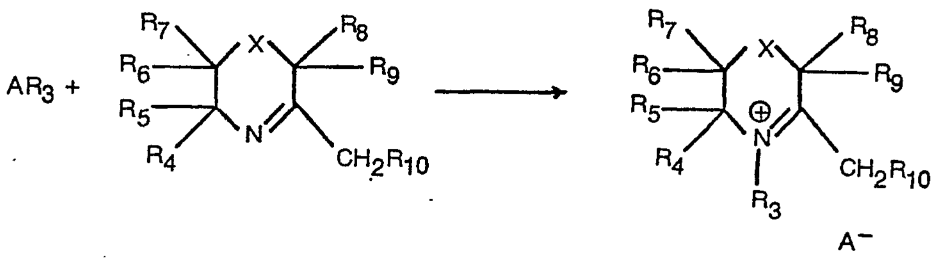

(V) (VI) (IV)

in der $R_3$ bis $R_{10}$ X und A die oben angeführten Konfigurationen darstellen.

10. Verfahren nach Anspruch 9, dadurch-gekennzeichnet, dass man zur Herstellung der Erzeugnisse, in denen X eine $CH_2$ Gruppe darstellt, ein Δ1-Piperidein der Formel (VI) herstellt, indem Li $CH_2$ $R_{10}$ auf einem Halogen ω-Nitril der Formel (VIII) nach folgendem Schema Kondensiert wird,

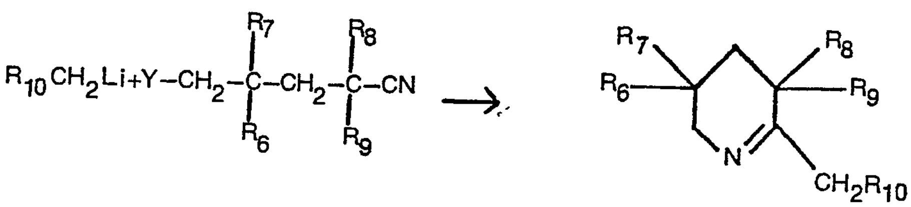

(VII) (VIII) (VI)

wobei die Gruppen $R_6$ bis $R_{10}$ die angegebenen Konfigurationen und Y Cl oder Br darstellen.

11. Verfahren nach Anspruch 9, dadurch gekennzeichnet dass man zur Herstellung der Erzeugnisse, in denen X ein S-Atom darstellt, ein Merkaptozeton der Formel (IX), nach dem untenstehenden Schema auf einem Aziridin der Formel (X) Kondensiert:

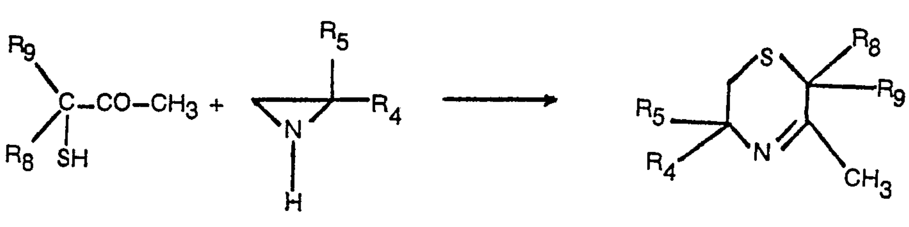

(IX) (X) (VI)

$R_4$, $R_5$, $R_8$ und $R_9$ entsprechen den oben angeführten Konfigurationen.

12. Photochromatische Verbindung, dadurch gekennzeichnet dass sie mindestens eine spiropyranische Verbindung nach einem der Ansprüche 1 bis 6 aufweist.

## Claims

1. Spiropyran compounds characterized by the fact that they have the general formula (I)

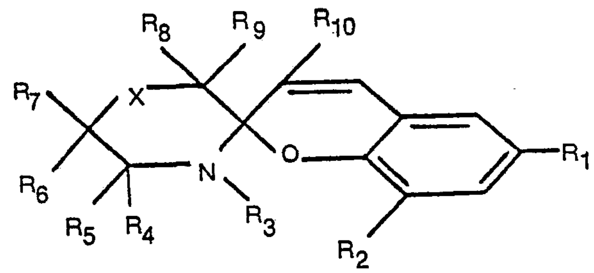

(I)

in which:

X represents the $CH_2$ group or a sulfur atom, $R_1$ and $R_2$ represent electron acceptor and/or donor groups,

$R_3$ is a linear or branched alkyl group having from 1 to 18 carbon atoms, an arylalkyl group in which the linear or branched alkyl group contains from 1 to 5 carbon atoms, or an alkenyl having from 2 to 10 carbon atoms,

$R_4$, $R_5$, $R_6$, $R_7$, $R_8$ and $R_9$ have any of the meanings indicated for $R_3$, represent a hydrogen atom or participate two by two in the formation of a saturated homocycle of 5 to 10 carbon atoms, either with the group present on the same carbon atom or with the group in ortho position,

$R_{10}$ represents a hydrogen atom if X=S while if X=$CH_2$ it represents a hydrogen atom, an alkyl group having 1 to 18 carbon atoms, an alkenyl group having 2 to 18 carbon atoms.

2. Compounds according to claim 1, characterised by the fact:

$R_1$ and $R_2$ are selected from the group consisting of $NO_2$, $-COC_6H_5$, CN, the halogens, the acyl substituents, the alkoxy substituents, the hydrogen atom, the alkyl or mercaptoalkyl groups, the ethers of formula $-CH_2OR$, thioethers of formula $CH_2SR$ and amines of formula $-CH_2NR_2$ in which R represents an alkyl group;

$R_3$ is selected from among the methyl, ethyl, isopropyl, n-butyl, benzyl or phenethyl substituents;

$R_4$, $R_5$, $R_6$, $R_7$, $R_8$ and $R_9$ have one of the meanings indicated for $R_3$, or represent, two by two, together with the group in ortho position a $-(CH_2)n$ group in which n is a number between 5 and 10, or a hydrogen atom;

$R_{10}$ represents a hydrogen atom if X=S and if X=$CH_2$ it represents a hydrogen atom or a methyl or ethyl substituent.

3. Compounds according claim 2 characterised by the fact

$R_1$ is represented by $NO_2$, $OCH_3$, $COC_6H_5$, I or Br;

$R_2$ is represented by $OCH_3$, $NO_2$, H, I or Br;

$R_4$ to $R_9$ are represented by a hydrogen atom or a methyl, ethyl, isopropyl, n-butyl, benzyl or phenethyl group.

4. Compounds according claim 3, characterised by the fact that

$R_3$ is represented by a methyl, ethyl or isopropyl groups $R_4$ to $R_9$ are selected from the group consisting of hydrogen and the methyl, ethyl and isopropyl groups.

5. Compounds according any one of claims 2 to 4, characterised by the fact that X represents $CH_2$, $R_4$ and $R_5$ hydrogen atoms, and $R_8$ and $R_9$ methyl substituents and $R_{10}$ a methyl or ethyl substituent.

6. Compounds according any one of claims 2 to 4, characterised by the fact that X represents a sulfur atom, $R_6$ and $R_7$ hydrogen atoms and $R_8$ methyl substituents and $R_{10}$ hydrogen.

7. A method of preparing spiropyran derivatives, characterised by the fact that it comprises condensing an orthohydroxy aromatic aldehyde of formula (III) with a quaternary salt of formula (IV)

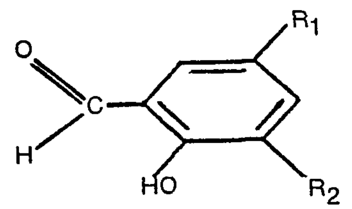

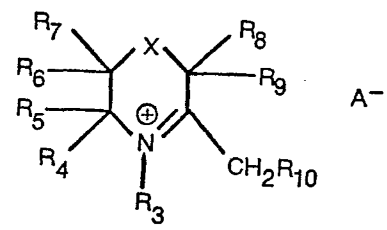

(III) (IV)

in which X and $R_1$ to $R_{10}$ have the meanings previously indicated and $A^-$ represents an anion selected from the group consisting of iodide, tosylate and methylsulfate anions.

8. A method according claim 7, characterised by the fact that the condensation is carried in basic medium.

9. A method according claim 7 or 8, characterised by the fact that the quaternary salt of formula (IV) is obtained by reacting an alkylation agent of formula (V) with a base of formula (VI) in accordance with the reaction:

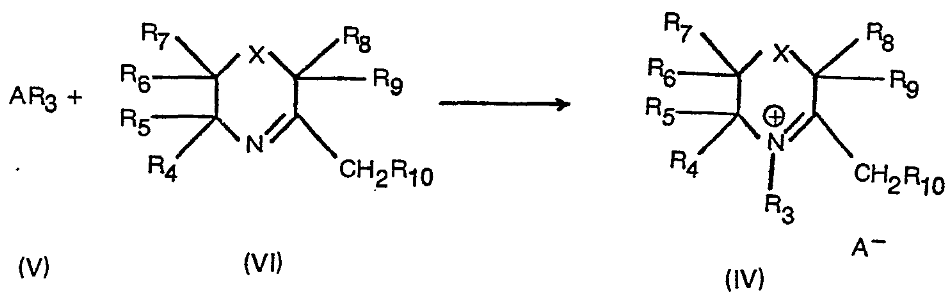

(V) (VI) (IV)

in which $R_3$ to $R_{10}$, X and A have the meanings indicated above.

10. A method according to claim 9, characterised by the fact that in order to obtain the products in which X represents the $CH_2$ group, Δ1-piperideine of formula (VI) is prepared by condensation of $LiCH_2R_{10}$ with an ω-halonitrile of formula VIII in accordance with the equation:

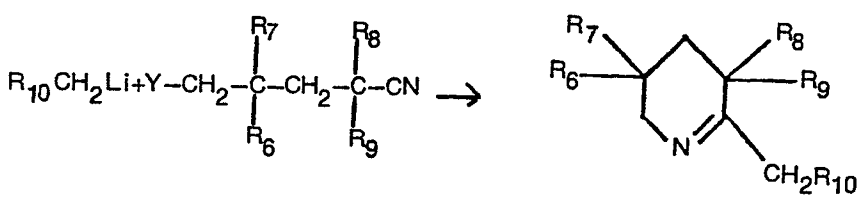

(VII) (VIII) (VI)

the groups $R_6$ to $R_{10}$ having the meanings indicated, and Y Cl or Br.

11. A method according to claim 9, characterised by the fact that in order to obtain products in which X represents a sulfur atom, a mercaptoketone of formula (IX) is reacted with an aziridine of formula (X), in accordance with the equation:

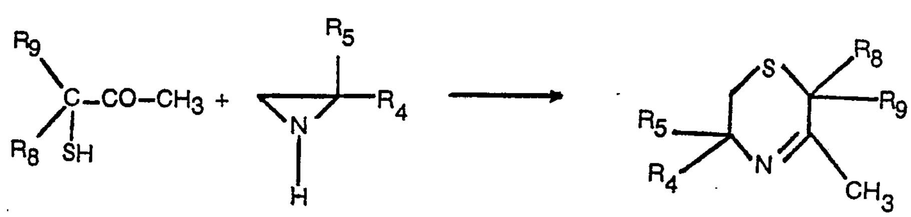

(IX) (X) (VI)

$R_4$, $R_5$, $R_8$ and $R_9$ having the meanings indicated above.

12. Photochromic composition, characterised by the fact that it comprises a spiropyran compound according to any one of claims 1 to 6.